# EUROPEAN PATENT SPECIFICATION

(11) **EP 1 796 682 B1**
(45) Date of publication and mention of the grant of the patent: **06.11.2013**
(21) Application number: 05800321.1
(22) Date of filing: 21.09.2005
(51) Int. Cl.: A61K 31/185, A61K 31/66, A61P 13/12, A61P 39/06

(54) **METHOD AND COMPOSITION FOR TREATING PATIENTS UNDERGOING KIDNEY DIALYSIS**
VERFAHREN UND ZUSAMMENSETZUNG ZUR BEHANDLUNG VON PATIENTEN, DIE EINER NIERENDIALYSE UNTERZOGEN WERDEN
METHODE ET COMPOSITION DE TRAITEMENT DE PATIENTS PAR DIALYSE RENALE

(30) Priority: 21.09.2004 US 945810
(43) Date of publication of application: 20.06.2007
(73) Proprietor: BIONUMERIK PHARMACEUTICALS, INC., San Antonio, Texas 78229 (US)
(72) Inventor: HAUSHEER, Frederick, H., Fair Oaks Ranch, TX 78015 (US)
(74) Representative: Lucas, Phillip Brian
(86) International application number: PCT/US2005/033631
(87) International publication number: WO 2006/034262

(56) References cited:
- EP-A- 1 323 440
- WO-A-2005/058300
- WO-A2-00/18881
- US-A- 5 661 188
- US-A- 6 031 006
- US-B1- 6 172 119
- URQUHART B L ET AL: "The effect of intravenous mesna (sodium 2-mercaptoethanesulfonic acid) on plasma homocysteine concentrations in hemodialysis patients." CAN J CLIN PHARMACOL, vol. 11(1), 1 June 2004 (2004-06-01), page E67, XP002475821
- SIU L L ET AL: "Use of mesna to prevent ifosfamide-induced urotoxicity." SUPPORTIVE CARE IN CANCER : OFFICIAL JOURNAL OF THE MULTINATIONAL ASSOCIATION OF SUPPORTIVE CARE IN CANCER MAR 1998, vol. 6, no. 2, March 1998 (1998-03), pages 144-154, XP002475822 ISSN: 0941-4355

## Description

### FIELD OF THE INVENTION

This invention relates to a compound and composition for use in treating patients undergoing hemodialysis or peritoneal dialysis, commonly referred to as kidney dialysis. An effective amount of a disulfide or thiol-containing compound is incorporated in the dialysis solution. The composition includes a dialysis solution and an effective amount of a thiol or reducible disulfide compound.

### BACKGROUND OF THE INVENTION

Hemodialysis, hereinafter referred to as kidney dialysis, or simply "dialysis," is a medical procedure that is performed on human patients (and also, on a smaller scale, pet animals), to remove accumulated waste and toxins from the blood in a similar manner to a functioning kidney. When a person or animal's kidneys cease to function properly due to one or more of a number of acute or chronic diseases or conditions (*e.g.*, diabetes, glomerulonephritis and hypertension are commonly recognized medical conditions that are associated with the development of renal failure), toxins accumulate in the bloodstream.

Failure to remove such accumulated waste and toxic compounds - primarily urea, uric acid and its analogues, and other nitrogenous compounds such as creatinine; and excess amounts of elements such as potassium, phosphorous, sodium, chloride and other minerals from the blood results in deterioration of body tissues and organ systems, which eventually results in death if untreated.

Dialysis may be performed in a hospital setting or clinic; or in some cases, the patient is trained to perform the procedure at home on an outpatient basis. Two primary types of dialysis are regularly performed - conventional hemodialysis and peritoneal dialysis. In conventional hemodialysis, the patient is connected (via an arteriovenous fistula, graft or by catheter) to a dialysis machine. The dialysis machine functions to pump the contaminated blood from the patient through a dialyzer, where the blood is filtered through a dialyzing solution, thereby lowering the concentration of accumulated waste (*e.g*., urea), and thence returned to the patient. Conventional hemodialysis usually takes between 3-6 hours and is normally performed at a clinic or hospital several times per week. Periodic testing is performed in patients undergoing hemodialysis to assess their medical need to undergo the procedure as well as to monitor the efficacy and timing of hemodialysis treatment.

In peritoneal dialysis, one end of a catheter is inserted into the patient's abdomen, with the catheter connected at its other end to a supply of dialysis solution. In a typical peritoneal dialysis exchange, dialysis solution is introduced into the patient's abdominal cavity through the catheter and allowed to remain there for a predetermined time period (called a "dwell"). During the period when the peritoneal cavity is filled with the solution, waste products and excess body fluids pass through the peritoneum, where they encounter the dialysis solution and are removed from the body when the solution is later drained from the body. The draining/filling process (the "cycle") is normally repeated several times daily, with a long dwell overnight. Periodic testing is performed to monitor the efficacy and timing of performing peritoneal dialysis.

Typical hemodialysis and peritoneal dialysis solutions contain dextrose (glucose), with the solution also including a quantity of salt and other dissolved minerals, and electrolytes as determined by the needs of each patient. The dialysis solution functions to increase osmotic pressure in the peritoneal cavity to cause maximum diffusion of excess fluids and waste products from the blood into the peritoneal cavity. The dialysis solution also serves to bind waste products for removal during the draining process, and to deliver necessary minerals and electrolytes to the body (many renal failure patients are placed on diets that shortchange necessary minerals, and must be ingested separately).

Mesna (sodium 2-mercaptoethene sulfonate) and dimesna (disodium 2,2'-dithiobis ethane sulfonate) are known therapeutic compounds that have heretofore demonstrated a wide variety of therapeutic uses. Both mesna and dimesna have shown protective effects against certain specific types of toxicity associated with the administration of cytotoxic drugs used to treat patients for various types of cancer.

In particular, mesna has been used with some success in mitigating the toxic effects of cytotoxic agents such as ifosfamide, oxazaphosphorine, melphalane, cyclophosphamide, trofosfamide, sulfosfamide, chlorambucil, busulfan, triethylene thiophosphamide, triaziquone, and others, as disclosed in U.S. Patent 4,220,660, issued September 2, 1980.

The improved toxicity profile of dimesna further underscores the usefulness of this compound.

Further, pharmacological profiles of each compound indicate that, if proper conditions are maintained, mesna and dimesna do not prematurely inactivate primary therapeutic drugs to a significant degree.

The molecular structures of both mesna and dimesna are shown below as Structure A and Structure B, respectively.

(A) HS-CH₂-CH₂-SO₃Na

(B) NaSO₃-CH₂-CH₂-S-S-CH₂-CH₂-SO₃Na

As shown, dimesna is a dimer of mesna, with the optimum conditions for oxidation occurring in the slightly basic (pH ~7.3), oxygen rich environment found in blood plasma. In mildly acidic, low oxygen conditions, in the presence of a reducing agent such as glutathione reductase, conditions prevalent in the kidneys, the primary constituent is mesna.

Mesna acts as a protective agent for a number of cytotoxic agents by substituting a nontoxic sulfhydryl moiety for a toxic hydroxy (or aquo) moiety. This action is particularly evidenced in the coadministration of mesna and oxazaphosphorine, and in the administration of dimesna along with certain platinum agents and/or taxanes.

Dimesna, as well as some analogues, have favorable toxicity profiles in mammalian species. Dimesna has been administered intravenously to mice and dogs in doses higher than the accepted oral LD50 for common table salt (3,750 mg/kg), with no adverse effects. In Phase I clinical trials, dimensa has been safely administered to humans in doses exceeding 40 g/m².

Mesna, and other analogues with free thiol moieties, constitute the more pharmacologically active reductive form of the two types of compounds described in this specification. These compounds manifest their activity by providing highly polar compositions of free thiol moieties for terminal substitution at locations where a terminal leaving group of appropriate configuration, usually a hydroxy, aquo or superoxide is located. Mesna also can form disulfide heteroconjugates with naturally occurring biochemicals that contain a free thiol moiety, such as cysteine, glutathione, homocysteine, and others.

Dimesna and other therapeutic disulfides can be reduced intracellularly by non-enzymatic thiol transfer reactions (SN2) that are mediated by the high intracellular concentration of glutathione and other physiological thiols, thereby generating higher concentrations of intracellular free thiols. The transient production of pharmacologically active free thiols act to scavenge the free radicals and other nucleophilic compounds often responsible for causing cell damage. Dimesna has also been shown to directly scavenge free radicals and may confer therapeutic benefit.

This profile is especially significant in explaining the success of dimesna in controlling and mitigating the toxic effects of platinum complex antitumor drugs. The mechanism for action in the case of cisplatin (*cis-*diammine dichloro platinum) is explained in United States Patent 5,789,000.

Mesna, dimesna, and analogues thereof, are synthesized from commonly available starting materials, using acceptable routes well known in the art. One such method involves the two-step, single pot synthetic process for making dimesna and like compounds of the following formula (I):

(I) R¹-S-R²;

wherein:
R¹ is hydrogen, -X-lower alkyl, or -X-lower alkyl-R³;
R² is -lower alkyl-R⁴;
R³ and R⁴ are each individually -SO₃M or -PO₃M₂;
X is absent or X is sulfur; and
M is an alkali metal.

As used herein, "lower alkyl" means an alkyl group of 1 to 8 carbon atoms.

The process essentially involves a two-step, single pot synthetic process, which results in the conversion of an alkyl sulfonate salt or acid or alkenyl sulfonate salt or acid to the desired formula (I) compound. The process in the case of mesna is a single step process that converts the alkyl or alkenyl sulfonate salt or acid to mesna, or a mesna derivative, by reaction with an alkali metal sulfide or with hydrogen sulfide.

If the desired end product is dimesna or a dimesna analogue, a two-step, single pot process is involved. Step 1 is as described above. Step 2 of the process is performed in the same reaction vessel as Step 1 without the need to purify or isolate the mesna formed during that step. Step 2 includes the introduction of oxygen gas into the vessel, along with an increase in pressure and temperature above ambient values, at least 20 pounds per square inch (psi) and at least 60°C. Dimesna or a derivative thereof is formed in essentially quantitative yield.

Other processes, well known and documented in the prior art, may be employed to make either mesna or dimesna, or derivatives and analogues thereof.

Urquhart et al, Can. J. Clin. Pharmacol., vol. 11(1), 1 June 2004, page e67, describes the effect of intravenous mesna on plasma homocysteine concentrations in hemodialysis patients.

### BRIEF SUMMARY OF THE INVENTION

One aspect of this invention is a compound for use in enhancing therapeutic effects of kidney dialysis in a mammalian patient. It involves the incorporation of a compound of formula (II), below, in a dialysis solution for a mammalian patient undergoing or about to undergo hemodialysis or peritoneal dialysis.
The aforementioned compound serves to increase the efficacy of the dialysis detoxification process. 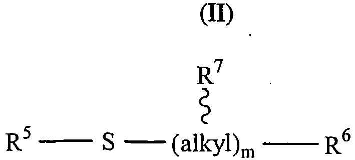 wherein:
R⁵ is hydrogen, lower alkyl or 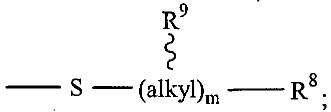
R⁶ and R⁸ are each individually -SO₃⁻M⁺, -PO₃²⁻M₂²⁺, or -PO₂S²⁻M²⁺;
R⁷ and R⁹ are each individually hydrogen, hydroxy or sulfhydryl;
each m is individually 1, 2, 3, 4, 5 or 6, with the proviso that if m is 1, then R⁷ is
hydrogen; and
M is hydrogen or an alkali metal ion; or
a pharmaceutically acceptable salt thereof.

Another aspect of this invention is a kidney dialysis composition comprising:
a dialysis solution; and
a compound of formula (II): 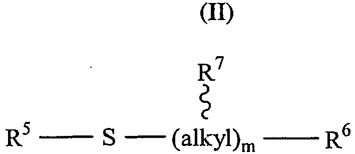 wherein:
   R⁵ is hydrogen, lower alkyl or 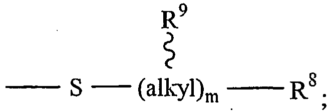
   R⁶ and R⁸ are each individually -SO₃⁻M⁺, -PO₃²⁻M₂²⁺, or -PO₂S²⁻M₂²⁺;
   R⁷ and R⁹ are each individually hydrogen, hydroxy or sulfhydryl;
   each m is individually 1, 2, 3, 4, 5 or 6, with the proviso that if m is 1, then R⁷
   hydrogen; and
   M is hydrogen or an alkali metal ion; or
a pharmaceutically acceptable salt thereof.

The compound of formula (II), when concomitantly utilized with the processes of hemodialysis or peritoneal dialysis, serves to increase the efficacy of the dialysis to aid in the detoxification of the patient's blood through the removal of various metabolic waste products which may have accumulated to toxic levels within the patient.

Pharmacologically effective amounts of the formula (II) compound to be incorporated in the dialysis solution according to the present invention are variable, and are dependent upon the individual patient's needs and/or upon the patient's response. The effective amount will also vary based upon the type of formula (II) compound to be incorporated, with disulfides usually requiring higher doses than thiols for effective treatment. Also, the type of dialysis procedure (i.e., hemodialysis or peritoneal dialysis) will affect the effective amount of incorporated formula (II) compound.

However, due to the excellent toxicity profile of the formula (II) compounds, large amounts of the compounds may be used without the risk of untoward side effects commonly associated with other drugs used to treat this condition.

Accordingly, it is an object of this invention to provide for a composition and compound for safely and effectively enhancing the efficacy of dialysis treatments.

Another object is to provide a composition and compound for enhancing performance of a dialysis procedure by incorporating a thiol or reducible disulfide in the dialysis solution for the patient undergoing or about to undergo dialysis treatment.

Other objects will become apparent upon a reading of the following description.

### DETAILED DESCRIPTION OF THE PREFERRED EMBODIMENTS

The preferred embodiments herein described are not intended to be exhaustive or to limit the invention to the precise form disclosed. They are chosen and described to explain the principles of the invention, and its application and practical use to best enable others skilled in the art to follow its teachings. The invention is defined in the appended claims.

The invention involves the incorporation of an effective amount of a formula (II) compound in a dialysis solution for a patient undergoing or about to undergo kidney dialysis treatment. The effective amount of the formula (II) compound will necessarily depend upon the individual patient's response. Since the formula (II) compounds are essentially nontoxic and cleared rapidly from the patient's body, large amounts of the formula (II) compound can normally be safely used.

A preferred formula (II) compound is one where:
R⁵ is hydrogen, lower alkyl or 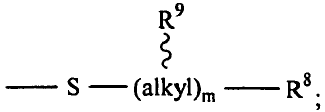
R⁶ and R⁸ are each individually -SO₃⁻M⁺, or -PO₃²⁻M₂²⁺;
R⁷ and R⁹ are each individually hydrogen or sulfhydryl;
each m is individually 1-5, with the proviso that if m is 1, then R⁷ is hydrogen; and
M is hydrogen or an alkali metal ion; or
a pharmaceutically acceptable salt thereof.

A more preferred formula (II) compound is one where:
R⁵ is hydrogen, lower alkyl or 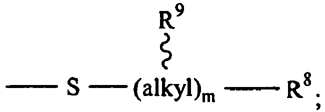
R⁶ and R⁸ are each individually -SO₃⁻M⁺, or -PO₃²⁻M₂²⁺;
R⁷ and R⁹ are each individually hydrogen;
each m is individually 2-4; and
M is hydrogen or an alkali metal ion; or
a pharmaceutically acceptable salt thereof.

Even more preferably, the formula (II) compound is a disulfide, as larger amounts of disulfide compounds may be used safely and effectively when compared to corresponding thiols and thioethers. The most preferred compound is disodium 2,2'-dithiobis ethane sulfonate (dimesna or Tavocept^{™}) used at a preferred dose of about 0.1 g to about 270 g added to each liter of fluid in the dialysis bag.

The formula (II) compound is preferably used in an amount of about 1 g/m² to about 80 g/m² of body surface area of the patient.

The formula (II) compound is added to the dialysis solution contained in the hemodialysis machine. As previously stated, the even more preferred compounds of formula (II) are the disulfides, and preferred effective amounts of these compounds to be utilized for hemodialysis range from about 0.1 g/L to about 270 g/L. The preferred effective amount is defined as a concentration of the formula (II) compound to the solution, ranging from about 0.1 mg/mL to about 270 mg/mL of solution.

Careful monitoring and analysis is performed regularly during hemodialysis, with additional compound used as needed. During peritoneal dialysis, most often performed at home by the patient and an assistant, monitoring may be performed as well to assess the effectiveness of the dialysis, and adjustments made in the treatment as necessary.

The composition according to the present invention includes any typical dialysis solution or one tailored to the needs of a particular patient. A dialysis solution may, by way of example but not of limitation, be comprised of: sterilized pyrogen-free water, dextrose, sodium, potassium, calcium, magnesium, and/or other cations, which may be present as a salt with chloride, acetate, lactate, and/or other anions, and the like. The exact composition of the dialysis solution is ordered by the physician and is dependent upon the patient's specific laboratory values and medical condition. A typical, preferred dialysis solution may be comprised of: dextrose in an amount of approximately 50 g/L, sodium chloride in an amount of approximately 5 g/L, sodium lactate in an amount of approximately 2.5 g/L, calcium chloride in an amount of approximately 1 g/L and magnesium chloride in an amount of approximately 0.5 g/L. The present invention relates to enhancing the performance of the dialysis solution by adding to it a compound of formula (II) in an amount of ranging from 0.1 g/l to 270 g/l.

The following hypothetical example is offered to further explain the invention.

### Example 1

### Continuous Ambulatory Peritoneal Dialysis (CAPD)

The patient about to undergo CAPD is fitted with an abdominal catheter. The open end of the catheter is connected to a 2 L bag of dialysis solution, which contains the following dissolved constituents: 100 g of dextrose; 10 g of sodium chloride; 5 g of sodium lactate; 2 g of calcium chloride; 1 g of magnesium chloride; and 40 g of dimesna.

The dialysis solution flows from the bag into the patient's abdomen and the bag is disconnected. The solution is allowed to "dwell" in the patient's abdomen for between 4 to 6 hours, then the catheter is reconnected to the bag and the solution is drained from the abdomen back into the bag. The process is repeated 2 to 4 times daily, and again just prior to the patient's going to sleep at night, whereupon the solution dwells in the abdomen for 6 to 8 hours. Spent dialysis solution may be analyzed from time-to-time in order to determine the efficiency of the dialysis, and to make modifications in treatment, if necessary.

## Claims

1. A compound of formula (II): 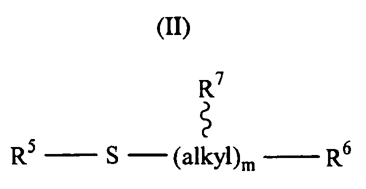 wherein:
R⁵ is hydrogen, an alkyl group of 1 to 8 carbon atoms or 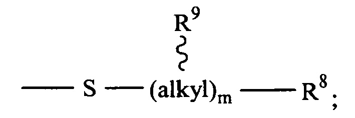
R⁶ and R⁸ are each individually -SO₃⁻M⁺, -PO₃²-M₂²⁺, or -PO₂S²⁻M₂²⁺;
R⁷ and R⁹ are each individually hydrogen, hydroxy or sulfhydryl;
each m is individually 1, 2, 3, 4, 5 or 6 with the proviso that if m is 1, then R⁷ is hydrogen; and
M is hydrogen or an alkali metal ion; or
a pharmaceutically acceptable salt thereof,
for use in enhancing therapeutic effects of kidney dialysis in a mammalian patient by aiding in the detoxification of the patient's blood through the removal of metabolic waste products, wherein a dialysis solution is to be used in the kidney dialysis and the formula (II) compound is to be administered to the patient undergoing or about to undergo kidney dialysis as a constituent of the dialysis solution.

2. The compound for use of claim 1, wherein the formula (II) compound is dimesna in an effective concentration in the dialysis solution of 0.1 g/L to 270 g/L.

3. The compound for use of claim 1, wherein the dialysis solution is to be administered to the patient while undergoing a hemodialysis or peritoneal dialysis procedure.

4. The compound for use of claim 1 or 3, wherein the formula (II) compound is dimesna in an effective concentration in the dialysis solution of 1 g/L to 200 g/L.

5. The compound for use of any preceding claim, wherein the dialysis solution is to be administered to the patient as a continuous ambulatory peritoneal dialysis solution.

6. The compound for use of any preceding claim, wherein the dialysis solution is a hemodialysis solution.

7. The compound for use of any one of claims 1, 3, 5 and 6, wherein in the formula (II) compound:
R⁵ is hydrogen, an alkyl group of 1 to 8 carbon atoms or 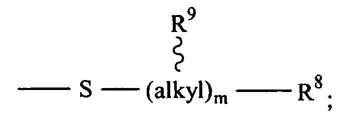
R⁶ and R⁸ are each individually -SO₃⁻M⁺, or -PO₃²⁻M₂²⁺;
R⁷ and R⁹ are each individually hydrogen or sulfhydryl;
each m is individually 1-5, with the proviso that if m is 1, then R⁷ is hydrogen; and
M is hydrogen or an alkali metal ion; or
a pharmaceutically acceptable salt thereof.

8. The compound for use of any one of claims 1, 3, 5 and 6, wherein in the formula (II) compound:
R⁵ is hydrogen, an alkyl group of 1 to 8 carbon atoms or 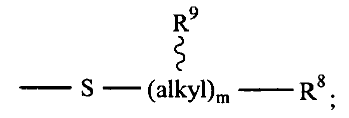
R⁶ and R⁸ are each individually -SO₃⁻M⁺, or -PO₃²⁺M₂²⁺;
R⁷ and R⁹ are each individually hydrogen;
each m is individually 2-4; and
M is hydrogen or an alkali metal ion; or
a pharmaceutically acceptable salt thereof.

9. The compound for use of claim 1 or 3, wherein the formula (II) compound is a disulfide or a pharmaceutically acceptable salt thereof.

10. The compound for use of any preceding claim, wherein the compound of formula (II) is to be administered in an amount of 1 g/m² to 80 g/m² of body surface area of the patient.

11. The compound for use of claim 1 or 3, wherein the compound of formula (II) is mesna.

12. The compound for use of claim 1 or 3, wherein the compound of formula (II) is dimesna.

13. A kidney dialysis composition comprising:
a dialysis solution suitable for administration to a patient about to undergo kidney dialysis, and
a compound of formula (II): 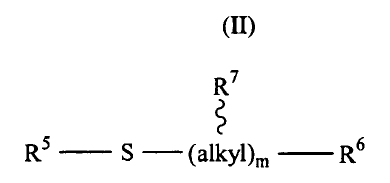 wherein:
R⁵ is hydrogen, an alkyl group of 1 to 8 carbon atoms or 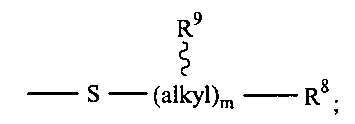
R⁶ and R⁸ are each individually -SO₃⁻M⁺, -PO₃²⁻M₂²⁺, or -PO₂S²-M₂²⁺;
R⁷ and R⁹ are each individually hydrogen, hydroxy or sulfhydryl;
each m is individually 1, 2, 3, 4, 5 or 6 with the proviso that if m is 1, then R⁷ is hydrogen; and
M is hydrogen or an alkali metal ion; or
a pharmaceutically acceptable salt thereof.

14. The kidney dialysis composition of claim 13, wherein in the formula (II) compound:
R⁵ is hydrogen, an alkyl group of 1 to 8 carbon atoms or 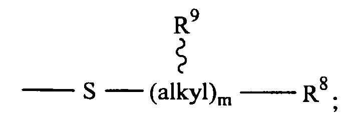
R⁶ and R⁸ are each individually -SO₃⁻M⁺, or -PO₃²⁻M₂²⁺;
R⁷ and R⁹ are each individually hydrogen or sulfhydryl;
each m is individually 1-5, with the proviso that if m is 1, then R⁷ is
hydrogen; and
M is hydrogen or an alkali metal ion; or
a pharmaceutically acceptable salt thereof.

15. The kidney dialysis composition of claim 13, wherein in the formula (II) compound:
R⁵ is hydrogen, an alkyl group of 1 to 8 carbon atoms or 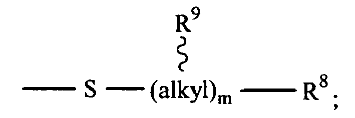
R⁶ and R⁸ are each individually -SO₃⁻M⁺, or -PO₃²⁻M₂²⁺;
R⁷ and R⁹ are each individually hydrogen;
each m is individually 2-4; and
M is hydrogen or an alkali metal ion; or
a pharmaceutically acceptable salt thereof.

16. The kidney dialysis composition of claim 13, wherein the formula (II) compound is mesna.

17. The kidney dialysis composition of claim 13, where the formula (II) compound is dimesna.

## Patentansprüche

1. Verbindung der Formel (II): 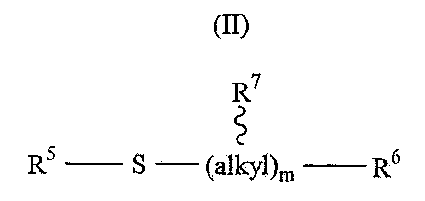 wobei:
R⁵ Wasserstoff, eine Alkylgruppe mit 1 bis 8 Kohlenstoffatomen oder 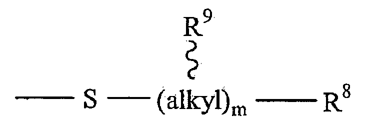 darstellt;
R⁶ und R⁸ jeweils einzeln -SO₃⁻M⁺, -PO₃²-M₂²⁺, oder -PO₂S²⁻M₂²⁺ darstellen;
R⁷ und R⁹ jeweils einzeln Wasserstoff, Hydroxy oder Sulfhydryl darstellen;
jedes m einzeln 1, 2, 3, 4, 5, oder 6 ist, mit der Maßgabe, dass, falls m 1 ist, R⁷ dann Wasserstoff darstellt; und
M Wasserstoff oder ein Alkalimetall-Ion darstellt; oder
ein pharmazeutisch verträgliches Salz derselben,
zur Verwendung im Verbessern der therapeutischen Wirkung einer Nierendialyse in einem Säugerpatienten, indem die Entgiftung des Blutes des Patienten, durch die Entfernung von Abfallprodukten des Stoffwechsels, gefördert wird, wobei eine Dialyselösung bei der Nierendialyse verwendet werden soll und die Verbindung der Formel (II) dem Patienten, der sich einer Nierendialyse unterzieht oder im Begriff ist, sich einer Nierendialyse zu unterziehen, als ein Bestandteil der Dialyselösung verabreicht werden soll.

2. Verbindung zur Verwendung nach Anspruch 1, wobei die Verbindung der Formel (II) Dimesna, in einer wirksamen Konzentration in der Dialyselösung von 0,1 g/L bis 270 g/L, ist.

3. Verbindung zur Verwendung nach Anspruch 1, wobei die Dialyselösung dem Patienten verabreicht werden soll, während er sich einer Hämo-Dialyse- oder Peritoneal-Dialyse-Prozedur unterzieht.

4. Verbindung zur Verwendung nach Anspruch 1 oder 3, wobei die Verbindung der Formel (II) Dimesna, in einer wirksamen Konzentration in der Dialyselösung von 1 g/L bis 200 g/L, ist.

5. Verbindung zur Verwendung nach einem der vorangegangenen Ansprüche, wobei die Dialyselösung dem Patienten als eine kontinuierliche, ambulante Peritoneal-Dialyselösung verabreicht werden soll.

6. Verbindung zur Verwendung nach einem der vorangegangenen Ansprüche, wobei die Dialyselösung eine Hämo-Dialyselösung ist.

7. Verbindung zur Verwendung nach einem der Ansprüche 1, 3, 5 und 6, wobei in der Verbindung der Formel (II):
R⁵ Wasserstoff, eine Alkylgruppe mit 1 bis 8 Kohlenstoffatomen oder 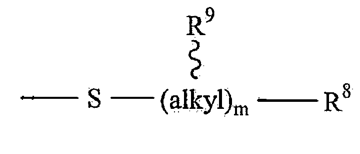 darstellt;
R⁶ und R⁸ jeweils einzeln -SO₃⁻M⁺ oder -PO₃²⁻M₂²⁺ darstellen;
R⁷ und R⁹ jeweils einzeln Wasserstoff oder Sulfhydryl darstellen;
jedes m einzeln 1-5 ist, mit der Maßgabe, dass, falls m 1 ist, R⁷ dann Wasserstoff darstellt; und
M Wasserstoff oder ein Alkalimetall-Ion darstellt; oder
ein pharmazeutisch verträgliches Salz derselben.

8. Verbindung zur Verwendung nach einem der Ansprüche 1, 3, 5 und 6, wobei in der Verbindung der Formel (II):
R⁵ Wasserstoff, eine Alkylgruppe mit 1 bis 8 Kohlenstoffatomen oder 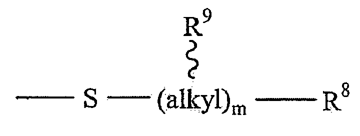 darstellt;
R⁶ und R⁸ jeweils einzeln -SO₃⁻M⁺ oder -PO₃²⁻M₂²⁺ darstellen;
R⁷ und R⁹ jeweils einzeln Wasserstoff darstellen;
jedes m einzeln 2-4 ist; und
M Wasserstoff oder ein Alkalimetall-Ion darstellt; oder
ein pharmazeutisch verträgliches Salz derselben.

9. Verbindung zur Verwendung nach Anspruch 1 oder 3, wobei die Verbindung der Formel (II) ein Disulfid oder ein pharmazeutisch verträgliches Salz desselben ist:

10. Verbindung zur Verwendung nach einem der vorangegangenen Ansprüche, wobei die Verbindung der Formel (II) in einer Menge von 1 g/m² bis 80 g/m² Körperoberfläehe des Patienten verabreicht werden soll.

11. Verbindung zur Verwendung nach Anspruch 1 oder 3, wobei die Verbindung der Formel (II) Mesna ist.

12. Verbindung zur Verwendung nach Anspruch 1 oder 3, wobei die Verbindung der Formel (II) Dimesna ist.

13. Nierendialysezusammensetzung, umfassend:
eine Dialyselösung, die zur Verabreichung an einen Patienten geeignet ist, der im Begriff ist, sich einer Nierendialyse zu unterziehen, und
eine Verbindung der Formel (II): 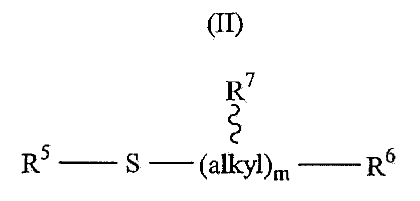 wobei:
R⁵ Wasserstoff, eine Alkylgruppe mit 1 bis 8 Kohlenstoffatomen oder 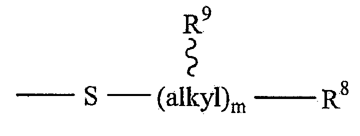 darstellt;
R⁶ und R⁸ jeweils einzeln -SO₃⁻M⁺, -PO₃²⁻M₂²⁺, oder -PO₂S²⁻M₂²⁺ darstellen;
R⁷ und R⁹ jeweils einzeln Wasserstoff, Hydroxy oder Sulfhydryl darstellen;
jedes m einzeln 1, 2, 3, 4, 5, oder 6 ist, mit der Maßgabe, dass, falls m 1 ist, R⁷ dann Wasserstoff darstellt; und
M Wasserstoff oder ein Alkalimetall-Ion darstellt; oder
ein pharmazeutisch verträgliches Salz derselben,

14. Nierendialysezusammensetzung nach Anspruch 13, wobei in der Verbindung der Formel (II):
R⁵ Wasserstoff, eine Alkylgruppe mit 1 bis 8 Kohlenstoffatomen oder 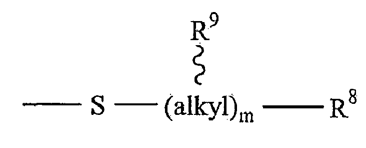 darstellt;
R⁶ und R⁸ jeweils einzeln -SO₃⁻M⁺ oder -PO₃²⁻M₂²⁺ darstellen;
R⁷ und R⁹ jeweils einzeln Wasserstoff oder Sulfhydryl darstellen;
jedes m einzeln 1-5 ist, mit der Maßgabe, dass, falls m 1 ist, R⁷ dann Wasserstoff darstellt; und
M Wasserstoff oder ein Alkalimetall-Ion darstellt; oder
ein pharmazeutisch verträgliches Salz derselben.

15. Nierendialysezusammensetzung nach Anspruch 13, wobei in der Verbindung der Formel (II):
R⁵ Wasserstoff, eine Alkylgruppe mit 1 bis 8 Kohlenstoffatomen oder 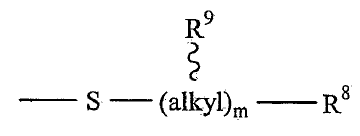 darstellt;
R⁶ und R⁸ jeweils einzeln -SO₃⁻M⁺ oder -PO₃²⁻M₂²⁺ darstellen;
R⁷ und R⁹ jeweils einzeln Wasserstoff darstellen;
jedes m einzeln 2-4 ist; und
M Wasserstoff oder ein Alkalimetall-Ion darstellt; oder
ein pharmazeutisch verträgliches Salz derselben.

16. Nierendialysezusammensetzung nach Anspruch 13, wobei die Verbindung der Formel (II) Mesna ist.

17. Nierendialysezusammensetzung nach Anspruch 13, wobei die Verbindung der Formel (II) Dimesna ist.

## Revendications

1. Composé de formule (II) : 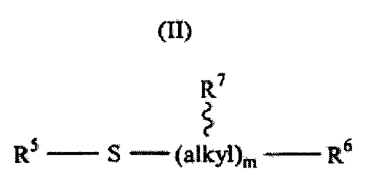 dans laquelle :
R⁵ est un hydrogène, un groupe alkyle de 1 à 8 atomes de carbone ou 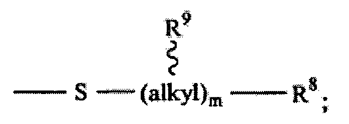
R⁶ et R⁸ sont chacun individuellement -SO₃⁻M⁺, -PO₃²M₂²⁺, ou -PO₂S²⁻M₂²⁺ ;
R⁷ et R⁹ sont chacun individuellement un hydrogène, un hydroxy ou sulfhydryle ;
chaque m vaut individuellement 1, 2, 3, 4, 5 ou 6 à condition que si m vaut 1, alors R⁷ est un hydrogène ; et
M est un hydrogène ou un ion de métal alcalin ; ou sel pharmaceutiquement acceptable de celui-ci,
à utiliser dans le renforcement des effets thérapeutiques de dialyse rénale chez un patient mammalien en facilitant la désintoxication du sang du patient via l'élimination de produits de déchet métabolique, où une solution de dialyse doit être utilisée dans la dialyse rénale et le composé de formule (II) doit être administré au patient subissant ou devant subir une dialyse rénale en tant que constituant de la solution de dialyse.

2. Composé pour utilisation selon la revendication 1, dans lequel le composé de formule (II) est du dimesna dans une concentration efficace dans la solution de dialyse de 0,1 g/L à 270 g/L.

3. Composé pour utilisation selon la revendication 1, dans lequel la solution de dialyse doit être administrée au patient pendant qu'il subit une procédure d'hémodialyse ou de dialyse péritonéale.

4. Composé pour utilisation selon la revendication 1 ou 3, dans lequel le composé de formule (II) est un dimesna dans une concentration efficace dans la solution de dialyse de 1 g/L à 200 g/L.

5. Composé pour utilisation selon l'une quelconque des revendications précédentes, dans lequel la solution de dialyse doit être administrée au patient en tant que solution de dialyse péritonéale ambulatoire continue.

6. Composé à utiliser selon l'une quelconque des revendications précédentes, dans lequel là solution de dialyse est une solution d'hémodialyse.

7. Composé pour utilisation selon l'une quelconque des revendications 1, 3, 5 et 6, dans lequel dans le composé de formule (II) :
R⁵ est un hydrogène, un groupe alkyle de 1 à 8 atomes de carbone ou 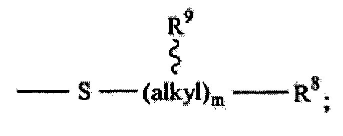
R⁶ et R⁸ sont chacun individuellement -SO₃⁻M⁺ ou -PO₃²M₂²⁺ ;
R⁷ et R⁹ sont chacun individuellement un hydrogène ou un sulfhydryle ;
chaque m est individuellement 1 à 5 à condition que si m vaut 1, alors R⁷ est un hydrogène ; et
M est un hydrogène ou un ion de métal alcalin ; ou sel pharmaceutiquement acceptable de celui-ci.

8. Composé pour utilisation selon l'une quelconque des revendications 1, 3, 5 et 6, dans lequel dans le composé de formule (II) :
R⁵ est un hydrogène, un groupe alkyle de 1 à 8 atomes de carbone ou 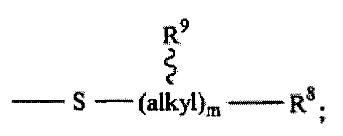
R⁶ et R⁸ sont chacun individuellement -SO₃⁻M⁺ ou -PO₃²M₂²⁺ ;
R⁷ et R⁹ sont chacun individuellement un hydrogène ;
chaque m vaut individuellement 2 à 4 ; et
M est un hydrogène ou un ion de métal alcalin ; ou sel pharmaceutiquement acceptable de celui-ci.

9. Composé pour utilisation selon la revendication 1 ou 3, dans lequel le composé de formule (II) est un disulfure ou un sel pharmaceutiquement acceptable de celui-ci.

10. Composé pour utilisation selon l'une quelconque des revendications précédentes, dans lequel le composé de formule (II) doit être administré dans une quantité de 1 g/m² à 80 g/m² de superficie corporelle du patient.

11. Composé pour utilisation selon la revendication 1 ou 3, dans lequel le composé de formule (II) est du mesna.

12. Composé à utiliser selon la revendication 1 ou 3, dans lequel le composé de formule (II) est du dimesna.

13. Composition de dialyse rénale comprenant :
une solution de dialyse adaptée pour être administrée à un patient sur le point de subir une dialyse rénale, et
un composé de formule (II) : 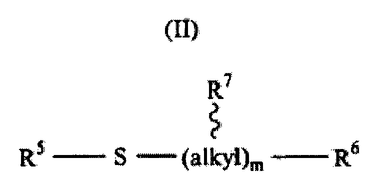 dans laquelle :
R⁵ est un hydrogène, un groupe alkyle de 1 à 8 atomes de carbone ou 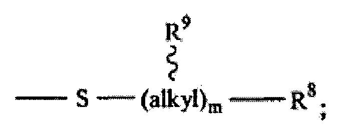
R⁶ et R⁸ sont chacun individuellement -SO₃⁻M⁺, -PO₃²M₂²⁺, ou -PO₂S²⁻M₂²⁺ ;
R⁷ et R⁹ sont chacun individuellement un hydrogène,
un hydroxy ou sulfhydryle ;
chaque m vaut individuellement 1, 2, 3, 4, 5 ou 6 à condition que si m vaut 1, alors R⁷ est un hydrogène et
M est un hydrogène ou un ion de métal alcalin ; ou sel pharmaceutiquement acceptable de celui-ci.

14. Composition de dialyse rénale selon la revendication 13, dans laquelle dans le composé de formule (II) :
R⁵ est un hydrogène, un groupe alkyle de 1 à 8 atomes de carbone ou 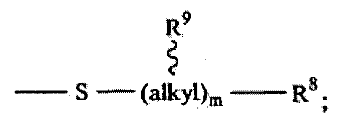
R⁶ et R⁸ sont chacun individuellement -SO₃⁻M⁺ ou -PO₃²M₂²⁺ ;
R⁷ et R⁹ sont chacun individuellement un hydrogène ou un groupe sulfhydryle ;
chaque m vaut individuellement 1 à 5 à condition que si m vaut 1, alors R⁷ est un hydrogène ; et
M est un hydrogène ou un ion de métal alcalin ; ou sel pharmaceutiquement acceptable de celui-ci.

15. Composition de dialyse rénale selon la revendication 13, dans laquelle dans le composé de formule (II) :
R⁵ est un hydrogène, un groupe alkyle de 1 à 8 atomes de carbone ou 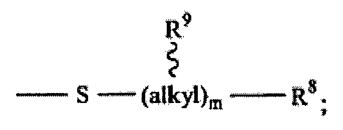
R⁵ et R⁸ sont chacun individuellement -SO₃⁻M⁺ ou -PO₃²M₂²⁺ ;
R⁷ et R⁹ sont chacun individuellement un hydrogène ;
chaque m vaut individuellement 2 à 4 ; et
M est un hydrogène ou un ion de métal alcalin ; ou sel pharmaceutiquement acceptable de celui-ci.

16. Composition de dialyse rénale selon la revendication 13, dans lequel le composé de formule (II) est du mesna.

17. Composition de dialyse rénale selon la revendication 13, dans lequel le composé de formule (II) est du dimesna.
